# EUROPEAN PATENT APPLICATION

(11) **EP 4 512 398 A1**
(43) Date of publication of application: **26.02.2025**
(21) Application number: 23193531.3
(22) Date of filing: 25.08.2023
(51) Int. Cl.: A61K 31/42, A61P 33/14

(54) **FLURALANER FOR USE IN METHODS OF TREATMENT AND PREVENTION OF SEA LICE INFESTATION IN FISH**

(71) Applicant: Bimeda Animal Health Limited, D18 K8Y4 Dublin 18 (IE); Bantry Marine Research Station Limited, D18 K8Y4 Dublin (IE)
(72) Inventor: TIERNEY, Dan, Dublin, A94 H2F7 (IE); MACLEOD RODGER, Hamish David, Galway, H91 F8XF (IE); O'NEILL, David, Dublin, D06 FX21 (IE); CAHALANE WILSON, Luke, Cork, P31 FP66 (IE)
(74) Representative: Purdylucey Intellectual Property

(57) **Abstract**

A compound of formula (2): 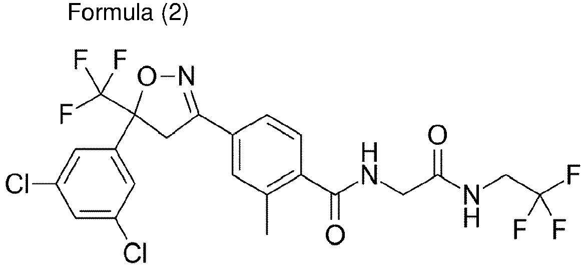 for use in the treatment or prevention of a sea lice infestation in a salmon, in which the compound is administered orally to the salmon.

## Description

### Field of the invention

The present invention relates to methods of treatment and prevention of sea lice infestation in fish.

### Background to the invention

The most significant health challenge for the global salmon farming industry remains sea lice and these crustacean parasites (*Lepeophtheirus salmonis* and *Caligus spp*.) cause skin damage to marine farmed salmonids which, if untreated, will result in increased susceptibility to other diseases, loss of growth, direct fish welfare problems and high mortality. This problem affects farmed salmon in Norway, Chile, Scotland, Canada, Faroe Islands and Ireland. There are a variety of treatment and management options which all can help to reduce the impact from lice. These include oral medicines, bath medicines, biological control through use of cleaner fish, mechanical methods to remove lice (high water pressure or brushes), high water temperature baths, net filters or screens, freshwater surface layers, etc., however, all methods and techniques have challenges and the need for access to effective medical treatments remains high. *Caligus elongatus* lice cause similar damage to *L. salmonis* on salmon with skin damage, reduced appetite, increased susceptibility to other diseases and eventually mortality if salmon are untreated. C. elongatus is less host specific than *L. salmonis* and has been reported on over 80 aquatic animal species and is a challenge for salmon farms in Norway, Scotland and Ireland.

It is an objective of the invention to overcome at least one of the above-referenced problems.

### Summary of the Invention

The objective is met by the provision of an isoxazoline-substituted benzoic acid amide for the treatment of sea lice infestation in salmonids and other species of fish. The Applicant has surprising discovered that oral administration of a single dose of the Active significantly reduces total lice numbers in salmon within one week of administration.

In a first aspect, the invention provides an isoxazoline-substituted benzoic acid amide (hereafter "Active") for use in the treatment or prevention of a sea lice infestation in an aquatic animal.

In any embodiment, the isoxazoline-substituted benzoid acid amide is Fluralaner or a functional variant of Fluralaner. Fluralaner has a structure of Compound (2) below:

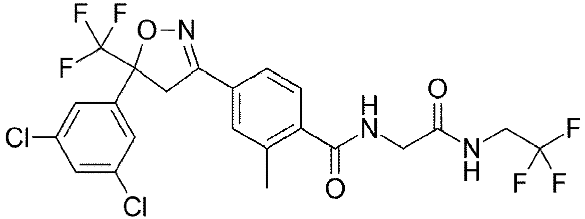

Fluralaner, functional variants of Fluralaner, and methods of synthesis of Fluralaner and functional variants are described in US Patent No: 7,662,972 and US Patent No: 8,389,738 (Nissan Chemical Industries, Ltd.).

In any embodiment, the Active is provided in an amorphous or a crystal form.

In any embodiment, the aquatic animal is a fish. Other aquatic animals include crustaceans such as prawns, crab and lobster.

In any embodiment, the fish is a farmed fish. Examples of farmed fish include salmonids, carp, catfish, trout, seabass, barramundi, grouper, sea bream, turbot and tilapia.

In any embodiment, the fish is a salmonid.

In any embodiment, the sea lice infestation is selected from a *Lepeophtheirus salmonis* infestation and a *Caligus spp.* infestation (e.g. *Caligus elongatus*).

In any embodiment, the aquatic animal to be treated is infested with at least 2, 5, 10, 15 or 20 sea lice.

In any embodiment, the method comprises administering a single dose of the Active to the aquatic animal.

In any embodiment, the treatment results in a reduction of total sea lice numbers in the treated aquatic animal of at least 50%, 60%, 70%, 80%, 90% or 95%, typically within one week of treatment.

In any embodiment, the Active is orally administered to the aquatic animal.

In any embodiment, the Active contained in an aquatic animal feed and the Active is administered to the aquatic animal by feeding the aquatic animal.

In any embodiment, the Active is orally administered to the aquatic animal at a dosage of 1-100 mg/kg body weight.

In any embodiment, the Active is orally administered to the aquatic animal at a dosage of 50-100 mg/kg body weight.

In any embodiment, the Active is orally administered to the aquatic animal at a dosage of 1-60 mg/kg body weight.

In any embodiment, the Active is orally administered to the aquatic animal at a dosage of 10-60 mg/kg body weight.

In any embodiment, the Active is orally administered to the aquatic animal at a dosage of 20-60 mg/kg body weight.

In any embodiment, the Active is orally administered to the aquatic animal at a dosage of 50-60 mg/kg body weight.

In another aspect, the disclosure provides a composition comprising an Active and a fish feed and optionally a binder.

In any embodiment, the composition comprises (w/w):
95.0 to 99.2 % fish feed (e.g. fish feed pellets);
0.2 to 2.5 % Active (e.g. Fluralaner); and
0.5 to 2.5 % binder (e.g. oil).

In any embodiment, the composition comprises (w/w):
97.0 to 99.2 % fish feed (e.g. fish feed pellets);
0.2 to 1.2 % Active (e.g. Fluralaner); and
0.5 to 1.0 % binder (e.g. oil).

In any embodiment, the fish feed is in a pelletised form.

In any embodiment, the binder is an oil.

In any embodiment, the Active is Fluralaner.

In any embodiment, the fish feed comprises vegetable components, fish meal, oil (usually fish oil) and usually fish protein concentrate.

Other aspects and preferred embodiments of the invention are defined and described in the other claims set out below.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an effective amount or a therapeutically effective amount of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition.

As used herein, the term "isoxazoline-substituted benzoic acid amide" refers the compounds claimed in US Patent No: 7,662,972, one example of which is the compound Fluralaner. Specific examples are compounds of formula (1): 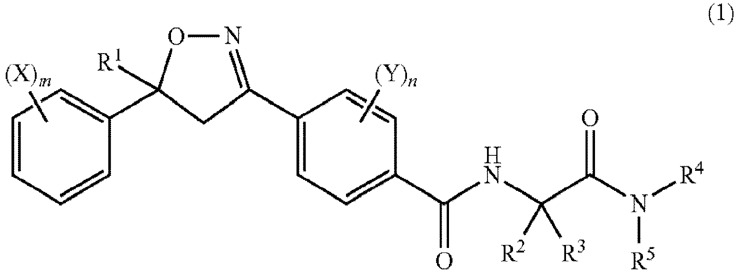
where X is a halogen atom, cyano, nitro, -SF₅, C₁₋₆ alkyl, C₁₋₄ haloalkyl, hydroxy(C₁₋₆)alkyl, hydroxy(C₁₋₆)haloalkyl, C₁₋₆ alkoxy(C₁₋₆)alkyl, C₁₋₆ haloalkoxy (C₁₋₆)alkyl, C₁₋₆ alkoxy(C₁₋₆)haloalkyl, C₁₋₆ haloalkoxy(C₁-₆)halo alkyl, C₃₋₈ cycloalkyl, C₃₋₈ halocycloalkyl, -OR⁶, -OSO₂R⁶, -S(O)R⁶, or -N(R⁸)R⁷, where when m is 2 or more, Xs are optionally the same as or different from each other,
Y is a halogen atom, cyano, nitro, C₁₋₆ alkyl, C₁₋₆ haloalkyl, C₁₋₆ alkoxy, C₁₋₆ haloalkoxy, C₁₋₆ alkylthio, C₁₋₆ haloalkylthio, C₁₋₆ alkylsulfonyl, C₁₋₆ haloalkylsulfonyl, or -N(R⁸)R⁷, where when n is 2 or more, Ys are optionally the same as or different from each other,
R¹ is a hydrogen atom, C₁₋₆ alkyl, C₁₋₆ haloalkyl, C₃₋₈ cycloalkyl, or C₃₋₈ halocycloalkyl,
R² is a hydrogen atom, cyano, C₁₋₆ alkyl, or C₁₋₆ haloalkyl,
R³ is a hydrogen atom or C₁₋₆ alkyl, or R³ together with R² optionally form a C₂₋₅ alkylene chain to form together with a carbon atom to which R³ is bonded a 3- to 6-membered ring, and at this time, the alkylene chain optionally contains one oxygen atom, one sulfur atom, or one nitrogen atom,
R⁴ is a hydrogen atom, C₁₋₆ alkyl, C₁₋₆ alkyl optionally substituted with R¹¹, C₁₋₆ haloalkyl, C₃₋₈ cycloalkyl, C₃₋₈ halocycloalkyl, C₃₋₆ alkenyl, C₃₋₆ haloalkenyl, C₃₋₆ alkynyl, phenyl, or phenyl substituted with (Z)t,
R⁵ is a hydrogen atom, C₁₋₆ alkyl, C₁₋₆ haloalkyl, C₃₋₈ cycloalkyl, C₃₋₈ halocycloalkyl, -CHO, C₁₋₆ alkylcarbonyl, C₁₋₆ haloalkylcarbonyl, C₁₋₆ alkoxycarbonyl, C₁₋₆ alkylsulfonyl, or C₁₋₆ haloalkylsulfonyl, or R⁵ together with R⁴ optionally form a C₂₋₆ alkylene chain to form together with a nitrogen atom to which R⁵ is bonded a 3- to 7-membered ring, and at this time, the alkylene chain optionally contains one oxygen atom, one sulfur atom, or one nitrogen atom and is optionally substituted with a halogen atom, a C₁₋₆ alkyl group, a C₁₋₆ alkoxy group, a formyl group, a C₁₋₆ alkylcarbonyl group, a C₁₋₆ alkoxycarbonyl group, an oxo group, or a thioxo group, R⁶ is C₁₋₆ alkyl, C₁₋₄ alkoxy(C₁₋₄)alkyl, C₁₋₆ haloalkyl, or C₁₋₄ haloalkoxy(C₁₋₄)haloalkyl,
R⁷ is C₁₋₆ alkyl, -CHO, C₁₋₆ alkylcarbonyl, C₁₋₆ haloalkylcarbonyl, C₁₋₆ alkoxycarbonyl, C₁₋₆ alkylsulfonyl, or C₁₋₆ haloalkylsulfonyl,
R⁸ is a hydrogen atom or C₁₋₆ alkyl,
R¹¹ is cyano, C₃₋₈ cycloalkyl, C₃₋₈ halocycloalkyl, -OR⁶, -S(O)R⁶, -N(R⁸)R⁷ phenyl, or phenyl substituted with (Z)t,
Z is a halogen atom, cyano, nitro, C₁₋₆ alkyl, C₁₋₆ haloalkyl, C₃₋₈ cycloalkyl, C₃₋₈ halocycloalkyl, -OR⁶, -OSO₂R⁶, -S(O)R⁶, or-N(R⁸)R⁷, where when t is 2 or more, Zs are optionally the same as or different from each other,
m is an integer of 0 to 5,
n is an integer of 0 to 4,
r is an integer of 0 to 2, and
t is an integer of 1 to 5.

As used herein, the term "fluralaner" refers to an isoxazoline-substituted benzoic acid amide of Compound (2) below:

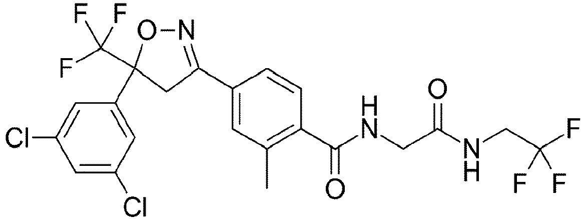

It is an authorised acaricide and insecticide, of the isoxazoline class, used in food producing poultry to treat and control red mite infestations (authorised in UK & Ireland under trade name Exzolt^{™} (from MSD)). The product is also widely prescribed and authorised as an oral treatment in cats and dogs. For poultry it is delivered via the drinking water and given in two-day treatments seven days apart. It acts by inhibiting parts of the parasite nervous system (GABA receptor & glutamate receptor) and has been shown in bio-assays to be effective against parasites with resistance to organophosphates, pyrethroids and carbamates. There are no previous reports of efficacy against sea lice, nor of pharmacokinetics or dynamics in salmon (or any fish species). It is effective against the feeding stages of the poultry mites and is considered worthy of further investigation for sea lice. Fluralaner, functional variants of Fluralaner, and methods of synthesis of Fluralaner and functional variants are described in US Patent No: 7,662,972, WO05/085216 and US Patent No: 8,389,738 (Nissan Chemical Industries, Ltd.).

As used herein the term "fish feed" refers to a composition, usually in a pelletised form, comprising vegetable components, fish meal, oil (usually fish oil) and usually fish protein concentrate. Fish oil is fat from fish parts or industrial fish (i.e. fish that is not intended for human consumption). Fish oil has a high content of the omega-3 fatty acids EPA and DHA. From one kilogram of industrial fish, around 80 grams of fish oil can be produced. Vegetable ingredients in fish feed are derived from plants like soy, sunflowers, rapeseed, corn, broad beans and wheat. The vegetable products are a source of protein, carbohydrates and fat. Fishmeal is produced by fish heads and other parts that are not used for human consumption. Fishmeal contains proteins and minerals. From one kilogram of industrial fish, around 230 grams of dry fishmeal can be produced. Fish protein concentrate is produced by scrapings from the consumer fishing industry. Fish feed also contains vitamins, minerals, pigments and amino acids. The antioxidant astaxanthin is added to salmon feed to boost the fish's immune system and to protect their tissue. It is also a source of vitamin A. Astaxanthin is the substance that gives salmon its red colour. Wild salmon get astaxanthin by eating crustaceans.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXPERIMENTAL

### Materials and Methods

A proof-of-concept experiment was carried out to determine the efficacy of fluralaner as a treatment for *L. salmonis* in farmed Atlantic salmon. Salmon infected with sea lice were divided evenly into three tanks, 41 fish per tank. Tank A acted as a control and no fluralaner was administered to the salmon, Tank B was given a low dog dose (25 mg/ kg of body weight) treatment and Tank C was given a high dog dose treatment (56 mg/ kg body weight). (See bravecto-epar-product-information attached for dosing rates). The powdered form of the drug was administered by mixing with standard aquaculture salmonid feed using fish oil as a binder. The experiment lasted for one week, between the 4/08/23 and the 11/08/23.

### Results

| | Tank A | Tank B | Tank C |
|---|---|---|---|
| Date | 04/08/2023 | 04/08/2023 | 04/08/2023 |
| Number of salmon sampled | 15 | 15 | 15 |
| Total lice | 341 | 387 | 350 |
| Average lice per fish | 22.7 | 25.8 | 23.3 |
| Date | 11/08/2023 | 11/08/2023 | 11/08/2023 |
| Number of salmon sampled | 15 | 15 | 15 |
| Total lice | 329 | 1 | 0 |
| Average lice per fish | 21.9 | 0.1 | 0 |

The results of the preliminary study indicate that fluralaner is highly effective in treating *L. salmonis* in Atlantic salmon. After one week, the total lice numbers on the salmon that received the treatment were significantly reduced, while the total lice numbers on the control tank remained high. There were no mortalities recorded during the trial period or since its discontinuation. Additionally, there appeared to be no obvious adverse effects on the salmon, and their behaviour can be considered normal. Samples have been retained for histology and residue testing.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A compound of formula (2): 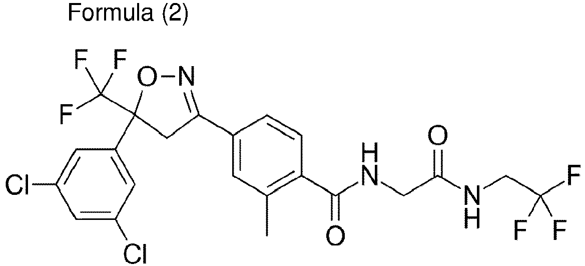 for use in the treatment or prevention of a sea lice infestation in a salmonid, in which the compound is administered orally to the salmonid.

2. A compound for use of Claim 1, in which the compound is orally administered to the salmonid at a dosage of 1-100 mg/kg body weight.

3. A compound for use of Claim 1, in which the compound is orally administered to the salmonid at a dosage of 20-60 mg/kg body weight.

4. A compound for use of any of Claims 1 to 3, in which the sea lice infestation is selected from a *Lepeophtheirus salmonis* infestation and a *Caligus spp.* infestation.

5. A compound for use of any of Claims 1 to 3, in which the sea lice infestation is selected from a *Lepeophtheirus salmonis* infestation and a *Caligus elongatus* infestation.

6. A compound for use of any of any preceding Claim, comprising a single dose of the compound.

7. A compound for use of any of any preceding Claim, in which the treatment results in a reduction of total sea lice numbers in the treated salmon of at least 80% within one week of treatment.

8. A compound for use of Claim 1, in which:
the salmonid is a farmed salmon;
the sea lice infestation is selected from a *Lepeophtheirus salmonis* infestation and a *Caligus elongatus* infestation;
the treatment comprises a single dose of the compound;
the compound is orally administered to the aquatic animal at a dosage of 20-60 mg/kg body weight; and
the treatment results in a reduction of total sea lice numbers in the treated salmon of at least 80% within one week of treatment.
